# EUROPEAN PATENT APPLICATION

(11) **EP 4 613 173 A1**
(43) Date of publication of application: **10.09.2025**
(21) Application number: 24175005.8
(22) Date of filing: 09.05.2024
(51) Int. Cl.: A61B 1/00, A61B 17/34

(54) **A SEALING DEVICE FOR USE IN ENDOSCOPY**

(30) Priority: 06.03.2024 US 202463561795 P
(71) Applicant: Marecik, Slawomir, Northfield IL 60093 (US)
(72) Inventor: Marecik, Slawomir, Northfield IL 60093 (US)
(74) Representative: Kancelaria Eupatent.pl Sp. z o.o.

(57) **Abstract**

A sealing device for use in endoscopy, having an elastic body (100) with an outer surface (110) and an inner surface (120) defining a through opening (121) extending from a leading end (101) to a trailing end (102) of the elastic body (100), wherein the outer surface has a round cross-section having a smallest outer diameter (d1c) in a middle portion, wherein the outer diameter increases from the middle portion towards each of the leading end (101) and the trailing end (102) of the sealing device, characterized in that the inner surface (120) has a round cross-section having a smallest inner diameter (d2c) in a middle portion, wherein the inner diameter increases from the middle portion towards each of the leading end (101) and wherein the trailing end (102) of the sealing device, and the body has a slit (130) extending from the leading end (101) towards the middle portion.

## Description

### TECHNICAL FIELD

The present invention relates to a sealing device for use in endoscopy, in particular in colonoscopy.

### BACKGROUND

Endoscopic medical procedures allow to visualize and examine interior surfaces of an organ or a cavity within a patient's body. They are performed by means of endoscopes, which are long, flexible tubes with a light source, a camera and various tools on a tip thereof.

Various endoscopic procedures may require insufflation of the cavity within which the endoscope operates, i.e. inflating the cavity with a gas (such as air or carbon dioxide) to provide more workroom. For efficient insufflation, it is preferable for the cavity to be relatively tightly sealed. Therefore, there is a need for appropriate sealing devices that can function as an inlet port to the patient's body and seal the space between the endoscopic tube and the patient's body at the point of entrance of the endoscope to the body.

Sealing may be particularly problematic during colonoscopic procedures, due to differences in sizes of anal canals of various patients.

A US patent US8968190B2 discloses a single incision surgical portal apparatus that comprises a housing member comprising an expandable material being adapted to transition the housing member between a compressed condition and an expanded condition in which an outer surface of the housing member is adapted for a substantial sealing relationship with tissue upon insertion of the housing member into the tissue for accessing an underlying tissue site, the housing member defining a longitudinal axis and having leading and trailing ends; and an inner member having a leading end and a trailing end, the leading end of the housing member and the leading end of the inner member being aligned along the longitudinal axis defined by the housing member such that the leading ends of the housing and inner members are coplanar, the inner member including at least one longitudinal port extending therethrough that is adapted for substantially sealed reception of a surgical object. This device, although made of a flexible material, has a predefined external dimensions, therefore it can be used to seal only openings of predefined sizes.

A Chinese utility model CN215348865U discloses a device for preventing air leakage of anus in colonoscopy, which comprises a body shaped as a truncated cone, a circular skin patch and a longitudinal straight crack. The longitudinal straight crack is used to facilitate mounting of the device on the colonoscopic tube. Similarly to the previously presented device, this one suffers from the same problem - although it is made of a flexible material, it has predefined external dimensions, therefore it can be used to seal only openings of predefined sizes. Moreover, its truncated cone shape does not provide efficient sealing from the internal side of the anal canal.

There is a need to provide an improved sealing device for use in endoscopy, in particular in colonoscopy, to allow efficient sealing of body openings of various sizes.

### SUMMARY OF THE INVENITON

The object of the invention is a sealing device for use in endoscopy, having an elastic body with an outer surface and an inner surface defining a through opening extending from a leading end to a trailing end of the elastic body, wherein the outer surface has a round cross-section having a smallest outer diameter in a middle portion, wherein the outer diameter increases from the middle portion towards each of the leading end and the trailing end of the sealing device. The inner surface has a round cross-section having a smallest inner diameter in a middle portion, wherein the inner diameter increases from the middle portion towards each of the leading end and the trailing end of the sealing device, and the body has a slit extending from the leading end towards the middle portion.

In use of the sealing device, the outer surface is configured for maintaining a sealing relationship with tissue upon insertion of the sealing device into the tissue for accessing an underlying tissue site. The internal surface is configured for maintaining a sealing relationship with an endoscopic tube at its middle portion.

Since the inner surface has a cross-sectional inner diameter increasing from a middle portion towards each of the leading end and the trailing end, the ends, are relatively flexible and can deform to adapt to the anal canal when the sealing device is installed.

Thereby, the sealing is achieved across a relatively long portion of the anal canal, at the ends of the sealing device, and not only in its most narrow portion, in the middle part of the sealing device.

The slit formed at least at the leading edge facilitates deformation of the sealing device once it is installed in the anal canal so as to more closely adhere to the inner walls of the anal canal, thereby achieving efficient sealing.

The body can be made of an elastic material having Shore A hardness between 20 and 50, preferably 30.

The body can be made of silicone, rubber or a thermoplastic elastomer.

The diameter of the external surface at the leading end can be smaller than the diameter of the external surface at the trailing end. This improves usability of the device as it is installed in the anal canal, where the leading portion to be located within the anal canal can be smaller than the portion that remains outside the patient's body. Alternatively, if the device is to be used with a patient having a large diameter of the anal canal, the sealing device can be used in a reverse manner, namely by inserting the larger diameter end into the anal canal.

Alternatively, the diameter of the external surface at the leading end can be equal to the diameter of the external surface at the trailing end. This simplifies manufacture of the sealing device and facilitates its use.

The outer surface and the inner surface may each have a shape of a hyperbolic hyperboloid. Such a shape facilitates optimal installation of the sealing device.

The outer surface and the inner surface may each have a cylindrical shape in the middle portion. This increases the sealing properties along the endoscopic tube and increases the coupling between the sealing device and the endoscopic tube.

The thickness of the body can be maximal at the middle portion and can decrease to a minimal thickness towards each of the end portions.

The slit in a side projection can be slanted with respect to the length direction of the sealing device. This facilitates deformation of the end portion of the sealing device when it is inserted into a narrow anal canal.

The slit may extend across the whole length of the sealing device, from its leading end to the trailing end.

The slit can be curved or v-shaped in a side projection, having an apex in the middle portion of the elastic body. This facilitates deformation of the elastic body.

The sealing device can be used in securing transrectal access during endoscopic examination such as colonoscopy, enteroscopy and (recto)sigmoidoscopy.

These and other features, aspects and advantages of the invention will become better understood with reference to the following drawings, descriptions and claims.

### BRIEF DESCRIPTION OF DRAWINGS

Various embodiments are herein described, by way of example only, with reference to the accompanying drawings, wherein:

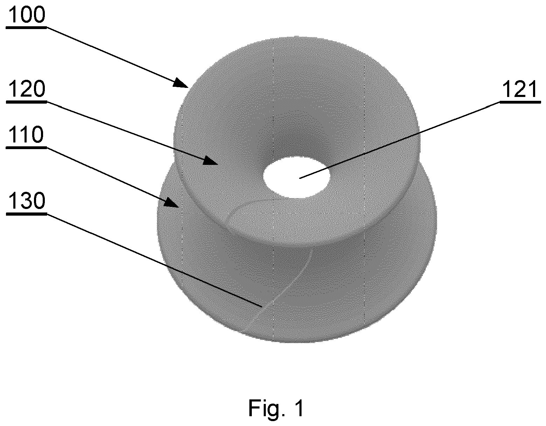

Fig. 1 shows an overall view of the sealing device;
Fig. 2 shows a side view of a first embodiment of the sealing device;
Fig. 3 shows a side view of the first embodiment of the sealing device with dimensional indicators;
Fig. 4 shows a side view of a second embodiment of the sealing device;
Fig. 5 shows a side view of a third embodiment of the sealing device;
Fig. 6 shows a side view of a fourth embodiment of the sealing device;
Fig. 7 shows a side view of a fifth embodiment of the sealing device;
Fig. 8 shows a side view of a sixth embodiment of the sealing device;
Fig. 9 shows a side view of a seventh embodiment of the sealing device;

### DETAILED DESCRIPTION

The following detailed description is of the best currently contemplated modes of carrying out the invention. The description is not to be taken in a limiting sense, but is made merely for the purpose of illustrating the general principles of the invention.

The sealing device according to the present invention is designed for use in endoscopy and in particular in colonoscopy. It has an elastic body 100 with an outer surface 110 and an inner surface 120. A through opening 121 extends from a leading end 101 to a trailing end 102 of the elastic body 100. The outer surface has a round cross-section that has a smallest outer diameter die in a middle portion and that increases from the middle portion towards each of the leading end 101 and the trailing end 102. Similarly, the inner surface 120 has a round cross-section having a smallest inner diameter d2c in a middle portion that increases from the middle portion towards each of the leading end 101 and the trailing end 102. The outer surface 110 and the inner surface 120 may have a cylindrical shape in the middle portion. An exemplary, but non-limiting three-dimensional shape that meet these criteria is a hyperbolic hyperboloid. The body has a slit 130 extending from the leading end 101 towards the middle portion of the elastic body, thereby facilitating deformation of the leading end 101.

The body 100 is made of an elastic material having Shore A hardness between 20 and 50, preferably 30, such as silicone, rubber or a thermoplastic elastomer.

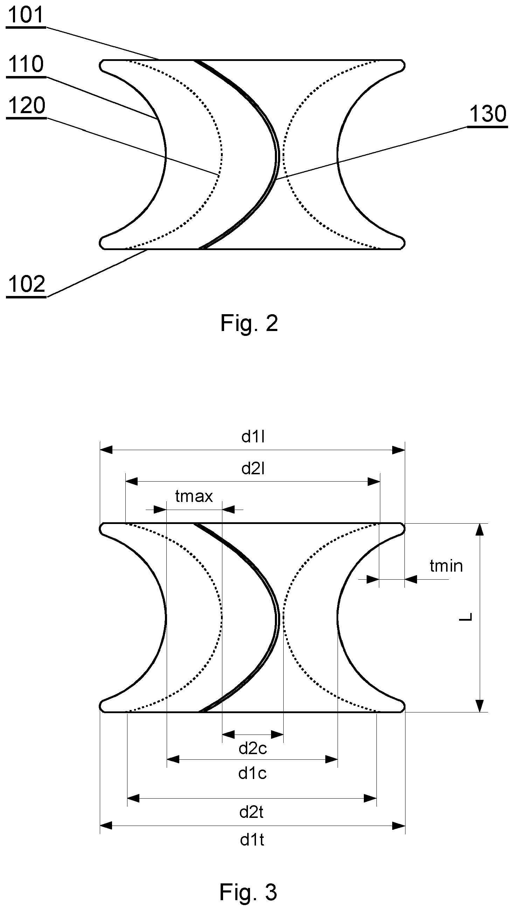

In a first embodiment of the sealing device, as shown in Fig. 2 and Fig. 3 wherein the dotted lines represent the contour of the inner surface 120, the diameter d1l of the external surface 110 at the leading end 101 equal to the diameter d1t of the external surface 110 at the trailing end 102.

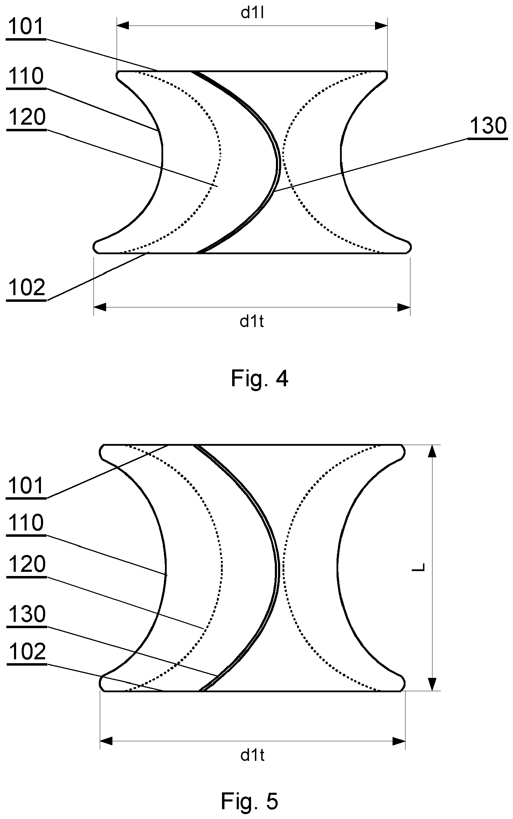

In turn, a second embodiment of the sealing device as shown in Fig. 4, differs from the first embodiment in that the diameter d1l of the external surface 110 at the leading end 101 is smaller than the diameter d1t of the external surface 110 at the trailing end 102.

The sealing device may have various proportions between the diameter and the length, such as the largest external diameter d1t may be larger than the length L of the device as shown in most embodiments or smaller than the length L of the device as shown in the third embodiment of Fig. 5.

The thickness of the body 100 is preferably maximal tmax at the middle portion and decreases to a minimal thickness tmin towards each of the end portions 101, 102. Preferable, but non-limiting dimensions of the sealing device are:
- external diameter d1l at the leading edge: from 30 to 70 mm, preferably 50 mm
- external diameter die at the middle portion: from 20 to 50 mm, preferably 30 mm
- external diameter dit at the trailing edge: from 30 to 70 mm, preferably 55 mm
- internal diameter d1c at the middle portion: from 9 to 15 mm, preferably 13 mm
- the maximum thickness tmax at the middle portion: from 5 to 11 mm, preferably 8,5 mm
- the minimum thickness tmin at the end portions can be from 1 to 10 mm, preferably 5 mm.

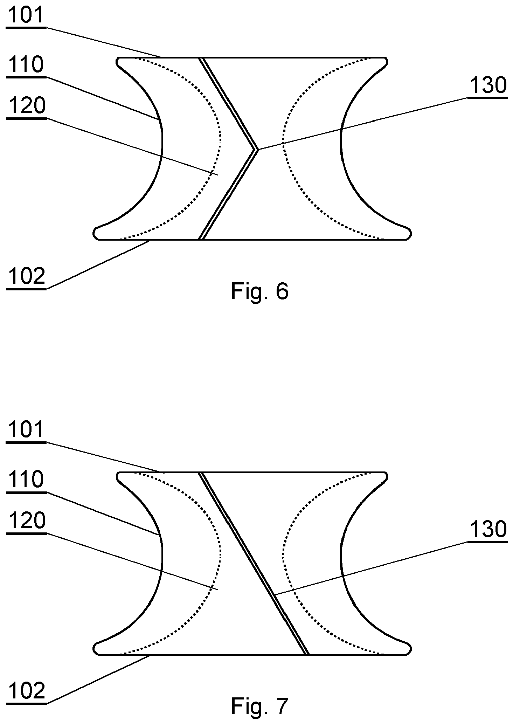

In the embodiments shown in Figs. 2-5, the slit 130 has a curved shape in a side projection, with an apex in the middle portion of the elastic body 100. However, the slit may have also a V-shaped shape in the side projection, such as shown in the fourth embodiment in Fig. 6, or a straight-line shape, slanted with respect to the length direction of the sealing device, as shown in the fifth embodiment in Fig. 7.

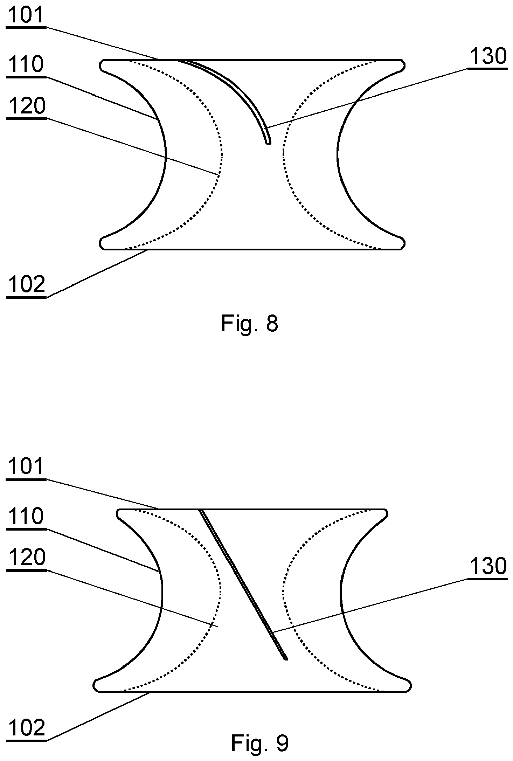

In the embodiments shown in Figs. 2-7, the slit 130 extends across the whole length of the sealing device, namely from its leading end 101 to the trailing end 102. However, the slit may also terminate before the middle portion of the elastic body, as shown in the sixth embodiment in Fig. 8, or beyond the middle portion of the elastic body, as shown in the seventh embodiment in Fig. 9.

The sealing device according to the present invention may be used to in securing transrectal access during endoscopic examination. By inserting the sealing device into the rectum, it fills the space between the surrounding tissue and the endoscope, allowing the gastrointestinal tract to better fill with gases during the examination. This allows better control over the examination environment which improves the quality of the examination and patient comfort. The sealing device is particularly suitable for use in examinations such as colonoscopy, enteroscopy and (recto)sigmoidoscopy.

While the invention has been described with respect to a limited number of embodiments, it will be appreciated that many variations, modifications and other applications of the invention may be made. Therefore, the claimed invention as recited in the claims is not limited to the embodiments described herein.

## Claims

1. A sealing device for use in endoscopy, having an elastic body (100) with an outer surface (110) and an inner surface (120) defining a through opening (121) extending from a leading end (101) to a trailing end (102) of the elastic body (100), wherein the outer surface has a round cross-section having a smallest outer diameter (d1c) in a middle portion, wherein the outer diameter increases from the middle portion towards each of the leading end (101) and the trailing end (102) of the sealing device, **characterized in that** the inner surface (120) has a round cross-section having a smallest inner diameter (d2c) in a middle portion, wherein the inner diameter increases from the middle portion towards each of the leading end (101) and wherein the trailing end (102) of the sealing device, and the body has a slit (130) extending from the leading end (101) towards the middle portion.

2. The sealing device according to claim 1, having the body (100) made of an elastic material having Shore A hardness between 20 and 50, preferably 30.

3. The sealing device according to any of previous claims, having the body (100) made of silicone, rubber or a thermoplastic elastomer.

4. The sealing device according to any of previous claims, wherein the diameter (d1l) of the external surface (110) at the leading end (101) is smaller than the diameter (d1t) of the external surface (110) at the trailing end (102).

5. The sealing device according to any of previous claims, wherein the diameter (d1l) of the external surface (110) at the leading end (101) is equal to the diameter (d1t) of the external surface (110) at the trailing end (102).

6. The sealing device according to any of previous claims, wherein the outer surface (110) and the inner surface (120) each have a shape of a hyperbolic hyperboloid.

7. The sealing device according to any of previous claims, wherein the outer surface (110) and the inner surface (120) each have a cylindrical shape in the middle portion.

8. The sealing device according to any of previous claims, wherein the thickness of the body (100) is maximal (tmax) at the middle portion and decreases to a minimal thickness (tmin) towards each of the end portions (101, 102).

9. The sealing device according to any of previous claims, wherein the slit (130) in a side projection is slanted with respect to the length direction of the sealing device.

10. The sealing device according to any of previous claims, wherein the slit (130) extends across the whole length of the sealing device, from its leading end (101) to the trailing end (102).

11. The sealing device according to any of previous claims, wherein the slit is curved or v-shaped in a side projection, having an apex in the middle portion of the elastic body (100).

12. The sealing device according to any of previous claims for use in securing transrectal access during endoscopic examination, in particular colonoscopy, enteroscopy or (recto)sigmoidoscopy.
